# EUROPEAN PATENT SPECIFICATION

(11) **EP 3 060 143 B1**
(45) Date of publication and mention of the grant of the patent: **06.01.2021**
(21) Application number: 14856589.8
(22) Date of filing: 24.10.2014
(51) Int. Cl.: A61K 31/337, A61M 25/04, A61M 25/10, A61L 29/14

(54) **MAINTENANCE OF BRONCHIAL PATENCY BY LOCAL DELIVERY OF CYTOTOXIC, CYTOSTATIC, OR ANTI-NEOPLASTIC AGENT**
BEKÄMPFUNG VON BRONCHIENLEIDEN DURCH LOKALE VERABREICHUNG ZYTOTOXISCHER, ZYTOSTATISCHER ODER ANTINEOPLASTISCHER MITTEL
CONSERVATION DE PERMÉABILITÉ BRONCHIQUE PAR ADMINISTRATION LOCALE D'AGENT CYTOTOXIQUE, CYTOSTATIQUE OU ANTI-NÉOPLASIQUE

(30) Priority: 25.10.2013 US 201361895779 P
(43) Date of publication of application: 31.08.2016
(73) Proprietor: Mercator Medsystems, Inc., Emeryville, California 94608 (US)
(72) Inventor: SEWARD, Kirk Patrick, San Francisco, CA 94114 (US)
(74) Representative: Gregson, Anna Louise
(86) International application number: PCT/US2014/062273
(87) International publication number: WO 2015/061748

(56) References cited:
- US-A1- 2005 042 295
- US-A1- 2005 042 295
- US-A1- 2005 137 611
- US-A1- 2006 263 434
- US-A1- 2006 263 434
- US-A1- 2008 276 935
- US-A1- 2008 300 571
- US-A1- 2010 145 307
- US-A1- 2010 145 307
- US-A1- 2010 202 979
- H. TSUKADA ET AL: "Macro and microscopic evluation of paclitaxel delivery in the airway with a novel endobronchial injectable drug delivery catheter", EUROPEAN RESPIRATORY JOURNAL, vol. 42, no. Suppl. 57, 1 September 2013 (2013-09-01), page P3760, XP055371295,

## Description

### BACKGROUND OF THE INVENTION

The present invention discloses generally medical methods and devices for the local delivery of therapeutic agents into the bronchial wall and other bodily lumen. More particularly, the present invention discloses intraluminal catheters with balloons having segments with different material moduli, which upon inflation improve apposition of tools against luminal structures, such as blood vessel walls or walls of other body lumens such as bronchi or the urethra. The present invention further discloses methods and systems for delivering agents adjacent to or within the encircling or encapsulating smooth muscle or connective tissue component of a conduit, vessel, or cavitary organ for the prophylaxis or treatment of disease.

Of particular interest to the present invention is the treatment of bronchial diseases. The bronchi in the respiratory tract conduct air into the lungs. Smooth muscle is present continuously around the bronchi. Many diseases of or around bronchial passageways can cause obstruction or narrowing of the bronchi. Cancer can be such a cause of narrowing for which medication delivered directly into the wall, whether anti-inflammatory, chemotherapeutic, paralytic, or otherwise may reduce the luminal narrowing and improve airflow without constriction.

Current estimates show that 226,000 people will be diagnosed in 2012 with lung and bronchial carcinoma in the U.S. About 160,000 of them are expected to die from this disease or its complications, such as obstructed airways. This disease impacts males and females with median age at death of 72 years. Malignant airway obstruction may potentially be treated with local, direct infusion of therapeutic agents into the bronchial wall and adventitia (the tissue between smooth muscle layers and cartilage) or directly into the tumor. Also, many other diseases of the bronchi, such as malignant airway obstruction, asthma, chronic bronchitis, arise in the sub-epithelial bronchial wall, and thus local treatment beyond the epithelium may be warranted.

In addition, other diseases of the airway may benefit from localized delivery of medication, fluids, bulking agents, biotherapeutics, or diagnostics. For example, tracheobronchial malacia may be treated with bulking or sclerosing agents to stiffen the airway wall and prevent expirational collapse of the airway. Mucous hypersecretion may be treated with agents to reduce the production of mucous, whether by killing or by altering mucous producing cells. Obstructive pulmonary diseases such as asthma or other non-cancerous obstructive disease may be caused by extensive localized edema. The reduction of edema may be possible with the delivery of agents to promote lymphangiogenesis and drain localized edema or toxin buildup from the airway tissues. Parasympathetic nerve responses or hyper-reactivity of airways to environmental stimulus may be reduced by the delivery of localized denervation agents.

Many current devices and methods, however, can be less than ideal for safely, reliably, and/or effectively delivering therapeutic agents to the bronchial wall. Drugs such as mitomycin, paclitaxel, and other anti-neoplastic agents, have been swabbed on the epithelial surface of the bronchus but retention of the swabbed drug may be less than ideal in at least some instances. Disadvantages of current clinical practice paradigms, include systemic and inhaled medications, may include overall side effects from increased absorption and decreased local concentration in the targeted area as many of these bronchial diseases are localized *in situ.* Thus, it would be desirable to provide improved medical devices, methods, and systems for the local delivery of therapeutic agents into the bronchial wall and other bodily lumens.

US 2010/145307 discloses intraluminal expandable catheters, which upon inflation improve apposition of tools against luminal structures. These catheters allow for intraluminal delivery of therapeutic agents in the body lumen through a microneedle, which can enter body lumen tissue, as well as the adventitia, media, or intima surrounding body lumens.

### BRIEF SUMMARY OF THE INVENTION

The invention is defined by the claims. Any subject-matter falling outside the scope of the claims is provided for information only. Any reference in the description to methods of treatment refer to the compounds, pharmaceutical compositions or medicaments of the present invention for use in the treatment of the human or animal body.

The present invention relates to a cytotoxic, cytostatic, or anti-neoplastic agent for use in maintaining patency in a bronchus of a patient, wherein the cytotoxic, cytostatic, or anti-neoplastic agent is to be delivered in a therapeutically beneficial amount effective to limit recurrent bronchial occlusion into one or more of a bronchial wall, submucosa, media, or adventitia of the bronchus, and wherein the agent is for delivery through a needle positioned through a wall of the bronchus so that an aperture of the needle is positioned at or beyond the bronchial adventitia, wherein the agent for delivery has a concentration in the range of 0.5 mg/mL to 1.5 mg/mL.

The present invention discloses catheters with a single balloon or other inflatable actuator which is inflated at a first pressure to unfurl or deploy a first portion of the balloon, where delivery of an additional inflation pressure or volume expands or otherwise deploys a second portion of the balloon wall to a size larger than or a configuration different than that achievable by inflation or unfurling of the first portion of the balloon wall alone. Multiple components may be combined into the same balloon or pressure component, such that one part of the wall is non-distensible and another part of the wall is compliant or elastomeric, such that a single inflation step, whether it involves volume or pressure, may be useful to activate both the non-distensible and compliant structures simultaneously or in series.

The present invention also discloses catheters and methods for deploying interventional tools in blood vessels and other body lumens. The interventional tools are typically needles which are penetrated into a luminal wall, but could be other structures such as atherectomy blades, optical fibers for delivering laser energy, mechanical abrasion and drilling components, and the like. The catheters comprise a catheter body having a proximal end and a distal end. The needle or other interventional tool is coupled to a distal portion of the catheter, and an inflatable structure is provided on or near the distal portion of the catheter body in order to advance the tool laterally relative to an axis of the catheter body. The inflatable structure may comprise two or more discrete regions which deform or inflate at different, typically successive inflation pressures. Usually, the regions will have different elasticities (where one may be substantially non-elastic or non-distensible), but in certain disclosures the regions could have identical elasticities where inflation of one or more of the regions below threshold pressure is prevented by tethers or other restraints which yield or break above said threshold pressure(s). By providing at least one non-distensible region, the non-distensible region can be fully inflated at relatively low pressures to a preselected size. If additional force or lateral displacement is needed, the inflatable structure can then be inflated beyond the first inflation threshold in order to expand one or more additional regions of the balloon, where the additional regions may have the same inflation characteristics or different inflation characteristics.

The regions of differing elasticity in the inflation structure can be achieved and fabricated in a variety of ways. In the disclosures below, the regions are formed in an edge-to-edge manner or along an overlapping border region using conventional masking and deposition techniques. It will be appreciated that the regions could also be formed by layering materials of differing elasticities, providing layers having different thicknesses, providing reinforcement fibers or materials which create regions of different elasticity within a matrix of the same material, providing tethers and other stretchable or breakable elements within regions of the inflation structure which yield or break when tension is applied above threshold levels, and the like.

The interventional tool may be mounted directly on the catheter body, but in the illustrated disclosures is mounted on the inflatable structure itself. It will be appreciated that more than one interventional tool may be mounted on the catheter, and that such multiple tools may be mounted directly on the catheter body, on the inflatable structure, or both.

By "non-distensible," it is meant that the material of the balloon will be inflatable from a lower profile or volume configuration to an expanded or higher profile or volume configuration. Once at the higher volume, expanded configuration, however, the material will no longer stretch or expand to any reasonable extent (typically less than 200% elongation in any direction prior to rupture) even though the inflation pressure can be raised significantly above the threshold pressure which achieves the higher volume inflation. By "elastomeric" it is meant that the material displays elasticity as more pressure is applied. Usually, there will be minimum or nominal stretching or expansion at or below the threshold pressure, but significant stretching and expansion at inflation pressures above the threshold pressure (typically at least 50% elongation in any direction prior to rupture, often at least 300% elongation in any direction prior to rupture, and usually at least greater than the elongation achievable by the non-distensible material prior to rupture). Additionally, the elastomeric materials will continue to stretch, usually in a nonlinear manner as pressure is increased above the threshold level.

The present invention further discloses methods for treating body lumens comprising introducing one or more interventional tools to the body lumen. An inflatable structure is inflated to a first pressure below a threshold pressure to advance the tool laterally to a first "maximum" distance which will not be exceeded so long as the pressure is maintained below the threshold pressure level. After inflation to the first pressure, if it is desired to further laterally advance the intervention tool, the inflatable structure may be inflated to a pressure which exceeds the first threshold pressure to further laterally advance the tool beyond the first maximum distance. The tool may be advanced to a second maximum distance, or alternatively may be incrementally advanced if the inflatable structure includes an elastic region which expands in linear or nonlinear proportion to the inflation pressure.

Such methods may be used to treat many diseases. In particular, such methods may be used to treat bronchial carcinoma or to maintain patency in a patient's bronchus which has had a bronchial carcinoma in the bronchus debulked (i.e., the bronchus has be recanalized). A catheter having the inflatable structure at its distal end can be positioned within the bronchus of the patient. A target region of one or more of a bronchial wall, submucosa, media, and adventitia can then be punctured at or adjacent a location of the debulked bronchial carcinoma with an injection needle disposed on the distal end of the catheter. And, an amount of a cytotoxic, cytostatic, or anti-neoplastic agent, such as paclitaxel, can be delivered to the target region through the injection needle. The delivered amount of cytotoxic, cytostatic, or anti-neoplastic agent may be effective to limit recurrent bronchial occlusion due to recurrence of the bronchial carcinoma by a therapeutically beneficial amount.

Such methods may also be used to treat diseases including asthma, chronic obstructive pulmonary disease (COPD), bronchitis, mucous hypersecretion, cystic fibrosis, or tracheobronchomalacia. As examples, in the case of tracheobronchomalacia, an airway has lost its rigidity. Agents such as bulking agents used in the common practice of plastic surgery (for example, Artefill®), sclerosing or fibrosing agents that can stiffen tissues, collagen, thermoset polymers, or the like can be delivered to the airway wall to provide stiffness without placing a stent in the lumen of the airway. In the case of bronchitis, localized antibiotics, anti-infectives, or steroids may be given to reduce the inflammation of the bronchus. With mucous hypersecretion or cystic fibrosis, agents may be delivered to reduce the hypersecretive process of mucous generation. With asthma or COPD, agents can be delivered to reduce edema, reduce hyperactivity of smooth muscle (such as by paralysis, lesioning, deadening), or reduce activity of sympathetic, parasympathetic or sensory nerves.

In a first aspect of the present disclosure, a medical device comprises a tubular member with a proximal and distal end, an involuted balloon at or near the distal end of the medical device with a working component embedded in the involuted segment, an ability to inflate the involuted balloon to deploy the working component, and a material with lower modulus than the involuted balloon material, affixed to and comprising part of the wall of the involuted structure, such that the lower modulus material may expand at a different rate and create an anchoring or opposing force to the working component. The material with lower modulus may be affixed in one or more ways to the material with higher modulus. In most cases, the lower modulus material resembles a "patch", or membrane structure, on the opposite side of the involuted structure from the working component.

In a second aspect of the present disclosure similar to the first aspect, the medical device comprises a tubular member with proximal and distal end, a working component at the distal end, and the requirement to place such working component asymmetrically against the wall of a body lumen. The attachment of the lower modulus "patch" to one side of the working component end structure allows for the asymmetric deployment of the working component via hydraulic or pneumatic pressurization of the lower modulus patch, or membrane, with respect to the higher modulus flexible but relatively non-distensible structure to which it is attached.

In a third aspect of the present disclosure, the working end of the tubular medical device may require particular positioning within a body lumen. Multiple low-modulus "patch", or membrane, structures may be affixed to a higher modulus structure such that the patches may be inflated individually or simultaneously in order to position the tip of the medical device appropriately within the body lumen.

In a fourth aspect of the present disclosure, the lower modulus "patch" or membrane structure and the higher modulus flexible but relatively non-distensible "anchor" structure meet at a joint that is formed between and consists only of the two materials constituting the patch and the anchor, respectively. The seal formed between the two materials at this joint is free from leakage below a particular amount of pressurization, and thus integrates the two materials to form one pressure vessel with wall components comprised of each material.

In a disclosure, the low-modulus material (the patch) is a flexible material such as silicone rubber or polydimethylsiloxane (PDMS). The high-modulus material (which can form the anchor to the patch or membrane) is a more flexible but relatively non-distensible polymer such as poly-paraxylylene (parylene N, C, or D). The low modulus material may be generally in a round and flat configuration, but may have more complex shape. The high modulus material is designed to have a "hole" in it approximately the size of the patch material, with some overlap to accommodate the attachment joint. The silicone patch, or membrane, and parylene flexible but relatively non-distensible material may be fixedly attached by polymeric encapsulation or polymeric adhesion, a process in which the parylene is vapor-deposited directly onto three substrates at once: a removable mold material adjacent to the silicone patch, the edge or border region of the silicone patch, and a removable (masking) material that protects the remainder of the silicone patch from being coated. When both removable materials are removed (e.g. by dissolution), the remaining structure is a parylene substrate with an affixed silicone patch, in which the joint formed between the two component structures consists only of the two constituent materials that comprise the individual components.

In the disclosure described above, the silicone patch may be on the back side of a folded balloon structure. The folded balloon structure is primarily comprised of parylene, but the patch comprises at least some of the surface area of the balloon. When the balloon is inflated, the flexible but relatively non-distensible structure unfolds, and then the elastomeric silicone expands due to pressurization. The flexible but relatively non-distensible parylene material unravels, but stretches much less than the silicone, thus forming the dual modulus balloon.

In a further disclosure, polymer vapor deposition may be used to form both the flexible but relatively non-distensible material component and a joint or interfacial region between the flexible component and the elastomeric component. Polymer vapor deposition of parylene or other suitable polymer typically begins with sublimation of a parylene dimer or other precursor at an elevated temperature in a low pressure chamber. The dimer vapor is then cleaved into monomer vapor as it travels through a higher temperature furnace. The monomer vapor travels into a deposition chamber, also held under vacuum, but at ambient temperature, at which point the monomer molecules rapidly lose energy and polymerize on surfaces within the deposition chamber. This process creates parylene coatings on components placed into the deposition chamber. Parylene coatings are usually nearly uniform, but thickness of the films varies based on the thermal properties of the system, the amount of dimer used, the intricacy of geometric surfaces placed into the deposition chamber, and the pressure at which the coating process is performed. By properly masking and creating layers, as described hereinafter, the flexible component and the elastomeric component may be joined as the flexible component is being formed. Other variables of the coating process also add to variance in the parylene coating characteristics.

In further disclosures, the lower-modulus material may be polyether block amide (Pebax), neoprene, Silastic®, chronoprene, C-flex, latex or other elastomeric materials.

In further disclosures, the higher-modulus material may be a thermoplastic polymer such as polyimide, polyethylene, polypropylene, polyethyl teraphthalate (PET), PTFE (Teflon©), PEEK, Tygon, nylon, acetal or other materials, including polymers, semiconductors, or metals, typically employed in the manufacture of medical devices and products.

In further disclosures, the attachment joint between the low modulus and high modulus material may be formed by polymer fusion at high temperature or pressure, by the use of adhesives such as cyanoacrylate, or by techniques employing surface preparation by electron bombardment of both materials and then placement of the materials in contact with each other. All of the above may be used to form leak-free seal joints between the low modulus and high modulus materials.

Aspects of the present disclosure also provide a method of maintaining bronchial patency in a bronchus of a patient. The method comprises delivering an amount of a therapeutic agent to tissue surrounding the bronchus. The delivered amount is effective to limit recurrent bronchial occlusion by a therapeutically beneficial amount. Delivery comprises injecting the amount of the therapeutic agent into one or more of a bronchial wall, submucosa, media, or adventitia of the bronchus.

The amount of the therapeutic agent is delivered to a site at or adjacent a cancerous tumor. The cancerous tumor may comprise a bronchia carcinoma, granuloma, fibrosis, or benign or malignant structure or narrowing. The amount of therapeutic agent may be delivered to the site at or adjacent a cancerous tumor which will typically have been debulked prior to delivery of the therapeutic agent. The delivered amount of therapeutic agent will typically be effective to prevent the recurrence of the cancerous tumor.

The therapeutic agent may be delivered through various steps. A needle is positioned through a wall of the bronchus so that an aperture of the needle is positioned at or beyond the bronchial adventitia. The needle may comprise a 35 to 45 gauge needle, preferably 45 gauge. The penetration of the therapeutic agent through the tissue may be confirmed by imaging either the therapeutic agent mixed with a diagnostic agent or by delivery of a diagnostic agent prior to the delivery of the therapeutic agent.

The method may further comprises steps of advancing a catheter into the bronchus and positioning the catheter adjacent a target region of the bronchial wall and adventitia before delivery of the therapeutic agent. A further step may include the expansion of an expandable element disposed on a distal end of the positioned catheter to cause a needle disposed on the expandable element to puncture the target region of the bronchial wall, submucosa, media, or adventitia before delivery of the therapeutic agent. The expandable element may comprise an inflatable balloon, and expansion of the expandable element may occur by inflation, preferably by air, but alternatively or in combination by saline or other buffers. The inflatable balloon may be inflated with 2 atmospheres of pressure without damaging the bronchus.

The therapeutic agent will comprise a cytotoxic, cytostatic, or anti-neoplastic agent. The therapeutic agent will often comprise paclitaxel. In some embodiments, the therapeutic agent comprises Abraxane®, a branded formulation of paclitaxel available from Celgene Corp. of Summit, New Jersey. The cytotoxic, cytostatic, or anti-neoplastic agent for delivery has a concentration in the range of 0. 5 mg/mL to 1 .5 mg/mL. Studies have been conducted that indicate the safety of a 1.5 mg/mL or less dosage and also strongly suggest both safety and efficacy for the local delivery of such a dosage to treat bronchial carcinomas and/or maintain airway patency by reducing their recurrence.

Other potential therapeutic agents include chemotherapeutic agents, specifically those cytotoxic agents traditionally used to treat cancer. Such agents may include, but are not limited to, alkylating agents such as busulfan, hexamethylmelamine, thiotepa, cyclophosphamide, mechlorethamine, uramustine, melphalan, chlorambucil, carmustine, streptozocin, dacarbazine, temozolomide, ifosfamide, and the like; anti-neoplastic agents such as mitomycin C and the like; anti-metabolites such as methotrexate, azathioprine, mercaptopurine, fludarabine, 5-fluorouracial, and the like; platinum-containing anti-cancer agents such as cisplatin, carboplatin and the like; anthracyclines such as daunorubicin, doxorubicin, epirubicin, idarubicin, mitoxantrone, and the like; plant alkaloids and terpenoids such as vincristine, vinblastine, vinorelbine, vindesine, podophyllotoxin, doclitaxel, and the like; topoisomerase inhibitors such as irinotecan, amsacrine, topotecan, etoposide, teniposide, and the like; antibody agents, such as rituximab, trastuzumab, bevacizumab, erlotinib, dactinomycin; finasteride; aromatase inhibitors; tamoxifen; goserelin; imatinib mesylate.

Other pulmonary diseases such as asthma, reactive airway disease, tachypnea, fibrotic lung diseases such as idiopathic pulmonary fibrosis and asbestosis, cystic fibrosis, interstitial lung disease, chemical pneumonitis, desquamative interstitial pneumonitis, nonspecific interstitial pneumonitis, lymphocytic interstitial pneumonitis, usual interstitial pneumonitis, idiopathic pulmonary fibrosis, pulmonary edema, aspiration, asphyxiation, pneumothorax, right-to-left shunts, left-toright shunts, respiratory failure, pneumonia, chronic obstructive pulmonary disease, emphysema, bronchitis, bronchopulmonary dysplasia, lung cancer, and the like may be treated by the devices, methods, and systems provided herein. Many of these diseases may involve the reduction of bronchial patency, for example, which can be treated by the devices, methods, and systems provided herein.

Aspects of the present disclosure also provide a method of maintaining patency in a patient's bronchus which has had a bronchial carcinoma in the bronchus debulked. A catheter is positioned within the bronchus of the patient. A target region of one or more of a bronchial wall, submucosa, media, and adventitia is punctured at or adjacent a location of the debulked bronchial carcinoma with an injection needle disposed on a distal end of the catheter. And, an amount of a cytotoxic, cytostatic, or anti-neoplastic agent is delivered to the target region through the injection needle. The delivered amount of cytotoxic, cytostatic, or anti-neoplastic agent is effective to limit recurrent bronchial occlusion due to recurrence of the bronchial carcinoma by a therapeutically beneficial amount.

The cytotoxic, cytostatic, or anti-neoplastic agent often comprises paclitaxel. In some embodiments, the cytotoxic, cytostatic, or anti-neoplastic agent for delivery may comprise Abraxane®, a branded formulation of paclitaxel. The target region is typically punctured with the injection needle by expanding an expandable element disposed on a distal end of the positioned catheter. The expandable element may comprise an inflatable balloon and expanding the expandable element may comprise inflating the balloon, preferably as with air, or alternatively or in combination with saline or other buffers. The cytotoxic, cytostatic, or anti-neoplastic agent for delivery typically has a concentration in the range of 0.5 mg/mL to 1 .5 mg/mL. The injection needle may comprise a 35 to 45 gauge needle, preferably a 45 gauge needle.

According to the claims, the present invention provides a therapeutic agent for use in maintaining patency in a bronchus of a patient. The therapeutic agent may be delivered in a therapeutically beneficial amount effective to limit recurrent bronchial occlusion. The therapeutic agent may be delivered into one or more of a bronchial wall, submucosa, media, or adventitia of the bronchus.

The therapeutically beneficial amount of the therapeutic agent may be delivered to a site at or adjacent a cancerous tumor. The cancerous tumor may comprise a bronchia carcinoma, granuloma, fibrosis, or benign or malignant structure or narrowing. The therapeutically beneficial amount of the therapeutic agent may be delivered to a site at or adjacent a cancerous tumor which may have been debulked prior to delivery of the therapeutic agent. The therapeutically beneficial amount of the therapeutic agent may be effective to prevent the recurrence of the cancerous tumor.

The therapeutically beneficial amount of the therapeutic agent may be delivered through a needle positioned through a wall of the bronchus so that an aperture of the needle is positioned at or beyond the bronchial adventitia. The needle may comprise a 35 to 45 gauge needle.

The penetration of the tissue by the therapeutic agent may be confirmed by imaging either the therapeutic agent mixed with a diagnostic agent or by delivery of a diagnostic agent prior to the delivery of the therapeutic agent.

The therapeutic agent may be delivered through a catheter advanced into the bronchus. The catheter may be positioned adjacent a target region of the bronchial wall and adventitia before delivery of the therapeutic agent. The catheter may comprise an expandable element disposed on a distal end thereof and a needle disposed on the expandable element. The expandable element may be expandable to cause the needle to puncture the target region of the bronchial wall, submucosa, media, or adventitia before delivery of the therapeutic agent. The expandable element may comprise an inflatable balloon. The inflatable balloon may be inflatable with 2 atmospheres of pressure without damaging the bronchus. The inflatable balloon may preferably be inflated with air or alternatively or in combination may be inflated with saline or other buffers.

The therapeutic agent may comprise a cytotoxic, cytostatic, or anti-neoplastic agent such as paclitaxel or Abraxane®. The therapeutic agent that is delivered has a concentration in the range of 0.5 mg/mL to 1 .5 mg/mL.

Aspects of the present disclosure also provide a system for use in maintaining patency in a bronchus of a patient. The system may comprise a therapeutic agent, a catheter configured to be placed within a bronchus of the patient, an expandable element disposed on a distal end of the catheter, an expandable element disposed on a distal end of the catheter, and an injection needle coupled to the expandable element. Expanding the expandable element may advance the injection needle in a direction transverse to a longitudinal axis of the catheter to puncture a target region of one or more of a bronchial wall, submucosa, media, and adventitia. The expandable element may comprise an inflatable balloon which may be inflated with air or alternatively or in combination inflated with saline or other buffers. When the needle has punctured the target region, the needle may deliver an amount of the therapeutic agent to the target region, and the amount may be effective to limit recurrent bronchial occlusion. The target region may be at or adjacent a location of a previously debulked bronchial carcinoma in the bronchus. The amount of therapeutic agent delivered may be effective to limit recurrent bronchial occlusion due to recurrence of the bronchial carcinoma by a therapeutically beneficial amount.

The therapeutic agent comprises a cytotoxic, cytostatic, or anti-neoplastic agent such as paclitaxel or Abraxane®. The therapeutic agent that is delivered has a concentration in the range of 0.5 mg/mL to 1 .5 mg/mL.

### BRIEF DESCRIPTION OF THE DRAWINGS

Fig. 1A is a schematic, perspective view of an intraluminal injection catheter suitable for use in the methods and systems of the present invention.
Fig. 1B is a cross-sectional view along line 1B-1B of Fig. 1A.
Fig. 1C is a cross-sectional view along line 1C-1C of Fig. 1A.
Fig. 2A is a schematic, perspective view of the catheter of Figs. 1A-1C shown with the injection needle deployed.
Fig. 2B is a cross-sectional view along line 2B-2B of Fig. 2A.
Fig. 3 is a schematic, perspective view of the intraluminal catheter of Figs. 1A-1C injecting therapeutic agents into an adventitial space surrounding a body lumen in accordance with the methods of the present invention.
Fig. 4 is a schematic, perspective view of another embodiment of an intraluminal injection catheter useful in the methods of the present invention.
Fig 5 is a schematic, perspective view of still another embodiment of an intraluminal injection catheter useful in the methods of the present invention, as inserted into one of a patient's body lumens.
Fig. 6 is a perspective view of a needle injection catheter useful in the methods and systems of the present invention.
Fig. 7 is a cross-sectional view of the catheter Fig. 6 shown with the injection needle in a retracted configuration.
Fig. 8 is a cross-sectional view similar to Fig. 7, shown with the injection needle laterally advanced into luminal tissue for the delivery of therapeutic or diagnostic agents according to the present invention.
Figs. 9A-9E are cross-sectional views of an exemplary fabrication process employed to create a free-standing low-modulus patch within a higher modulus anchor, framework or substrate.
Figs. 10A-10D are cross-sectional views of the inflation process of an intraluminal injection catheter useful in the methods of the present invention.
Figs. 11A-11C are cross-sectional views of the inflated intraluminal injection catheter useful in the methods of the present invention, illustrating the ability to treat multiple lumen diameters.
Fig. 12 is a diagram of representative conduits of the human respiratory system, including the bronchi B and trachea T, around which agents may be delivered according to the present invention.
Fig. 13A is a chart of paclitaxel plasma concentrations over 7-days for various local dosages of paclitaxel in a porcine study.
Fig. 13B is a graph of paclitaxel plasma concentrations (AUC Curves) over 7-days for various local dosages of paclitaxel in a porcine study.
Fig. 14 is a graph of average paclitaxel concentration at 7 days over 4 cm of bronchial tissue centered around an injection site (2 cm distal and 2 cm proximal).
Fig. 15 is a graph of paclitaxel plasma levels in individual bronchial segments up to 6 cm from the injection Site (between 1d = 1 cm distal from the injection site and 1p = 1 cm proximal).

### DETAILED DESCRIPTION OF THE INVENTION

By way of example, the first eight figures illustrate a needle injection catheter that can benefit from the dual modulus balloon offered by the present disclosure.

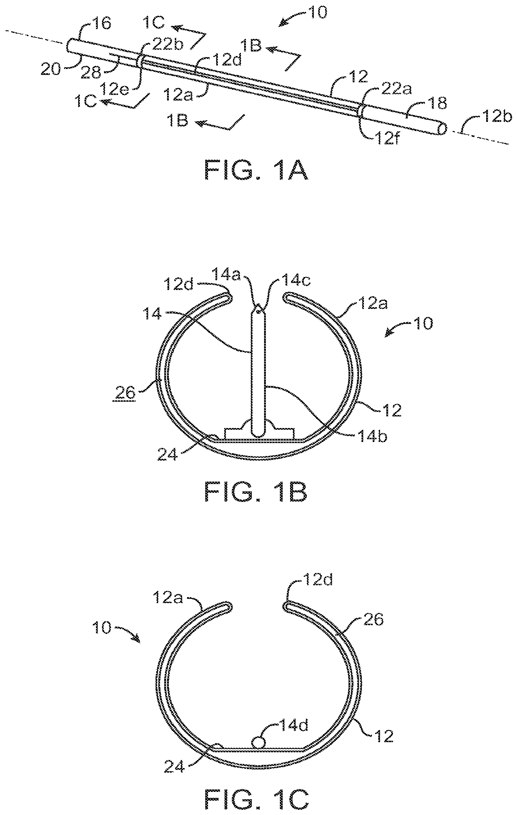

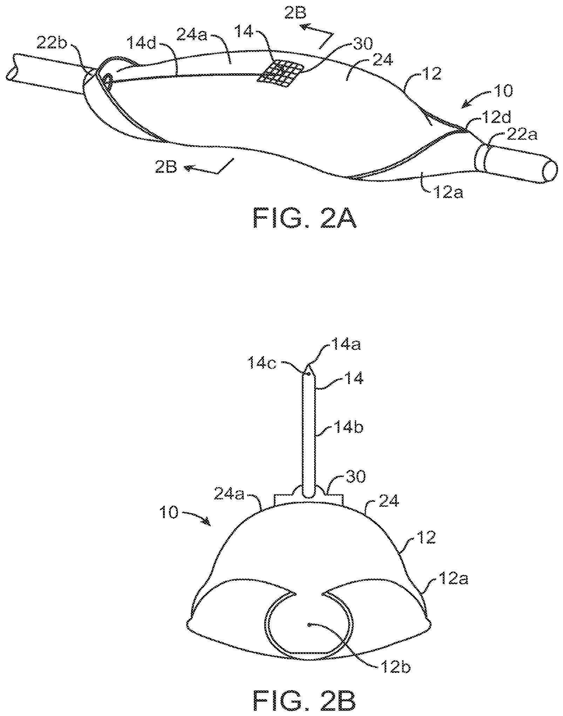

As shown in Figs. 1A-2B, a microfabricated intraluminal catheter 10 includes an actuator 12 having an actuator body 12a and central longitudinal axis 12b. The actuator body more or less forms a C-shaped outline having an opening or slit 12d extending substantially along its length. A microneedle 14 is located within the actuator body, as discussed in more detail below, when the actuator is in its unactuated condition (furled state) (FIG. 1B). The microneedle is moved outside the actuator body when the actuator is operated to be in its actuated condition (unfurled state) (FIG. 2B).

The actuator may be capped at its proximal end 12e and distal end 12f by a lead end 16 and a tip end 18, respectively, of a therapeutic catheter 20. The catheter tip end serves as a means of locating the actuator inside a body lumen by use of a radio opaque coatings or markers. The catheter tip also forms a seal at the distal end 12f of the actuator. The lead end of the catheter provides the necessary interconnects (fluidic, mechanical, electrical or optical) at the proximal end 12e of the actuator.

Retaining rings 22a and 22b are located at the distal and proximal ends, respectively, of the actuator. The catheter tip is joined to the retaining ring 22a, while the catheter lead is joined to retaining ring 22b. The retaining rings are made of a thin, on the order of 10 to 100 microns (µm), substantially flexible but relatively non-distensible material, such as Parylene (types C, D or N), or a metal, for example, aluminum, stainless steel, gold, titanium or tungsten. The retaining rings form a flexible but relatively non-distensible substantially "C"- shaped structure at each end of the actuator. The catheter may be joined to the retaining rings by, for example, a butt-weld, an ultra sonic weld, integral polymer encapsulation or an adhesive such as an epoxy.

The actuator body further comprises a central, expandable section 24 located between retaining rings 22a and 22b. The expandable section 24 includes an interior open area 26 for rapid expansion when an activating fluid is supplied to that area. The central section 24 is made of a thin, semi-flexible but relatively non-distensible or flexible but relatively non-distensible, expandable material, such as a polymer, for instance, Parylene (types C, D or N), silicone, polyurethane or polyimide. The central section 24, upon actuation, is expandable somewhat like a balloon-device.

The central section is capable of withstanding pressures of up to about 200 psi upon application of the activating fluid to the open area 26. The material from which the central section is made of is flexible but relatively non-distensible or semi-flexible but relatively non-distensible in that the central section returns substantially to its original configuration and orientation (the unactuated condition) when the activating fluid is removed from the open area 26. Thus, in this sense, the central section is very much unlike a balloon which has no inherently stable structure.

The open area 26 of the actuator is connected to a delivery conduit, tube or fluid pathway 28 that extends from the catheter's lead end to the actuator's proximal end. The activating fluid is supplied to the open area via the delivery tube. The delivery tube may be constructed of Teflon© or other inert plastics. The activating fluid may be a saline solution or a radio-opaque dye.

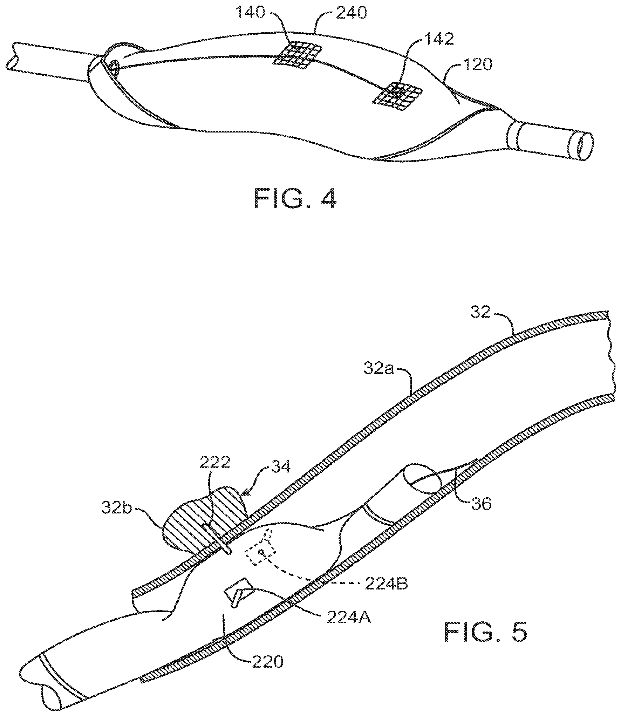

The microneedle 14 may be located approximately in the middle of the central section 24. However, as discussed below, this is not necessary, especially when multiple microneedles are used. The microneedle is affixed to an exterior surface 24a of the central section. The microneedle is affixed to the surface 24a by an adhesive, such as cyanoacrylate. Alternatively, the microneedle maybe joined to the surface 24a by a metallic or polymer mesh-like structure 30 (See FIG. 4), which is itself affixed to the surface 24a by an adhesive. The mesh-like structure may be-made of, for instance, steel or nylon.

The microneedle includes a sharp tip 14a and a shaft 14b. The microneedle tip can provide an insertion edge or point. The shaft 14b can be hollow and the tip can have an outlet port 14c, permitting the injection of a pharmaceutical or drug into a patient. The microneedle, however, does not need to be hollow, as it may be configured like a neural probe to accomplish other tasks.
As shown, the microneedle extends approximately perpendicularly from surface 24a. Thus, as described, the microneedle will move substantially perpendicularly to an axis of a lumen into which has been inserted, to allow direct puncture or breach of body lumen walls.

The microneedle further includes a pharmaceutical or drug supply conduit, tube or fluid pathway 14d which places the microneedle in fluid communication with the appropriate fluid interconnect at the catheter lead end. This supply tube may be formed integrally with the shaft 14b, or it may be formed as a separate piece that is later joined to the shaft by, for example, an adhesive such as an epoxy.

The needle 14 may be a 30-gauge, or smaller, steel needle. Alternatively, the microneedle may be microfabricated from polymers, other metals, metal alloys or semiconductor materials. The needle, for example, may be made of Parylene, silicon or glass. Microneedles and methods of fabrication are described in U.S. Application Serial No. 09/877,653, filed June 8, 2001, entitled "Microfabricated Surgical Device", assigned to the assignee of the subject application, the entire disclosure of which is incorporated herein by reference.

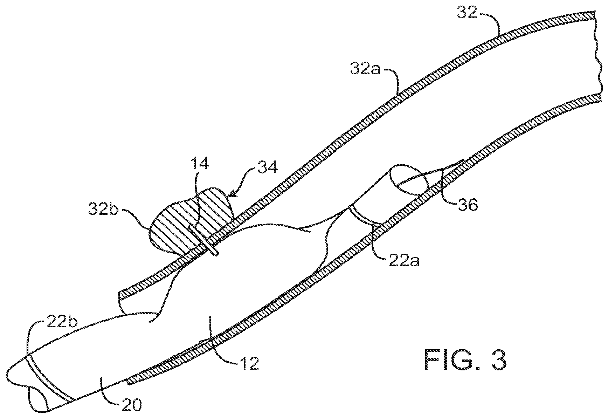

The catheter 20, in use, is inserted through an opening in the body (e.g. for bronchial or sinus treatment) or through a percutaneous puncture site (e.g. for artery or venous treatment) and moved within a patient's body passageways 32, until a specific, targeted region 34 is reached (see FIG. 3). The targeted region 34 may be the site of tissue damage or more usually will be adjacent the sites typically being within 100 mm or less to allow migration of the therapeutic or diagnostic agent. As is well known in catheter-based interventional procedures, the catheter 20 may follow a guide wire 36 that has previously been inserted into the patient. Optionally, the catheter 20 may also follow the path of a previously-inserted guide catheter (not shown) that encompasses the guide wire.

During maneuvering of the catheter 20, well-known methods of fluoroscopy or magnetic resonance imaging (MRI) can be used to image the catheter and assist in positioning the actuator 12 and the microneedle 14 at the target region. As the catheter is guided inside the patient's body, the microneedle remains unfurled or held inside the actuator body so that no trauma is caused to the body lumen walls.

After being positioned at the target region 34, movement of the catheter is terminated and the activating fluid is supplied to the open area 26 of the actuator, causing the expandable section 24 to rapidly unfurl, moving the microneedle 14 in a substantially perpendicular direction, relative to the longitudinal central axis 12b of the actuator body 12a, to puncture a body lumen wall 32a. It may take only between approximately 100 milliseconds and five seconds for the microneedle to move from its furled state to its unfurled state.

The ends of the actuator at the retaining rings 22a and 22b remain fixed to the catheter 20. Thus, they do not deform during actuation. Since the actuator begins as a furled structure, its so-called pregnant shape may exist as an unstable buckling mode. This instability, upon actuation, may produce a large-scale motion of the microneedle approximately perpendicular to the central axis of the actuator body, causing a rapid puncture of the body lumen wall without a large momentum transfer. As a result, a microscale opening is produced with very minimal damage to the surrounding tissue. Also, since the momentum transfer is relatively small, only a negligible bias force is required to hold the catheter and actuator in place during actuation and puncture.

The microneedle aperture, in fact, travels with such force that it can enter body lumen tissue 32b as well as the adventitia, media, or intima surrounding body lumens. Additionally, since the actuator is "parked" or stopped prior to actuation, more precise placement and control over penetration of the body lumen wall are obtained.

After actuation of the microneedle and delivery of the agents to the target region via the microneedle, the activating fluid is exhausted from the open area 26 of the actuator, causing the expandable section 24 to return to its original, furled state. This also causes the microneedle to be withdrawn from the body lumen wall. The microneedle, being withdrawn, is once again sheathed by the actuator.

Various microfabricated devices can be integrated into the needle, actuator and catheter for metering flows, capturing samples of biological tissue, and measuring pH. The device 10, for instance, could include electrical sensors for measuring the flow through the microneedle as well as the pH of the pharmaceutical being deployed. The device 10 could also include an intravascular ultrasonic sensor (IVUS) for locating vessel walls, and fiber optics, as is well known in the art, for viewing the target region. For such complete systems, high integrity electrical, mechanical and fluid connections are provided to transfer power, energy, and pharmaceuticals or biological agents with reliability.

By way of example, the microneedle may have an overall length of between about 200 and 3,000 microns (µm). The interior cross-sectional dimension of the shaft 14b and supply tube 14d may be on the order of 20 to 250 um, while the tube's and shaft's exterior cross-sectional dimension may be between about 100 and 500 µm. The overall length of the actuator body may be between about 5 and 50 millimeters (mm), while the exterior and interior cross-sectional dimensions of the actuator body can be between about 0.4 and 4 mm, and 0.5 and 5 mm, respectively. The gap or slit through which the central section of the actuator unfurls may have a length of about 4-40 mm, and a cross-sectional dimension of about 50-500 µm. The diameter of the delivery tube for the activating fluid may be about 100 µm. The catheter size may be between 1.5 and 15 French (Fr).

Variations of the invention include a multiple-buckling actuator with a single supply tube for the activating fluid. The multiple-buckling actuator includes multiple needles that can be inserted into or through a lumen wall for providing injection at different locations or times.

For instance, as shown in FIG. 4, the actuator 120 includes microneedles 140 and 142 located at different points along a length or longitudinal dimension of the central, expandable section 240. The operating pressure of the activating fluid is selected so that the microneedles move at the same time. Alternatively, the pressure of the activating fluid may be selected so that the microneedle 140 moves before the microneedle 142.

Specifically, the microneedle 140 is located at a portion of the expandable section 240 (lower activation pressure) that, for the same activating fluid pressure, will buckle outwardly before that portion of the expandable section (higher activation pressure) where the microneedle 142 is located. Thus, for example, if the operating pressure of the activating fluid within the open area of the expandable section 240 is two pounds per square inch (psi), the microneedle 140 will move before the microneedle 142. It is only when the operating pressure is increased to four psi, for instance, that the microneedle 142 will move. Thus, this mode of operation provides staged buckling with the microneedle 140 moving at time t₁, and pressure p₁, and the microneedle 142 moving at time t₂ and p₂, with t₁, and p₁, being less than t₂ and p₂, respectively.

This sort of staged buckling can also be provided with different pneumatic or hydraulic connections at different parts of the central section 240 in which each part includes an individual microneedle.

Also, as shown in FIG. 5, an actuator 220 could be constructed such that its needles 222 and 224A move in different directions. As shown, upon actuation, the needles move at angle of approximately 90° to each other to puncture different parts of a lumen wall. A needle 224B (as shown in phantom) could alternatively be arranged to move at angle of about 180° to the needle 224A.

The above catheter designs and variations thereon, are described in published U.S. Patent Application Nos. 2003/005546 and 2003/0055400. Co-pending Application No. 10/350,314, assigned to the assignee of the present application, describes the ability of substances delivered by direct injection into the adventitial and pericardial tissues of the heart to rapidly and evenly distribute within the heart tissues, even to locations remote from the site of injection. An alternative needle catheter design suitable for delivering the therapeutic or diagnostic agents of the present invention will be described below. That particular catheter design is described and claimed in co-pending application 10/397,700 (Attorney Docket No. 021621-001500 US), filed on March 19, 2003.

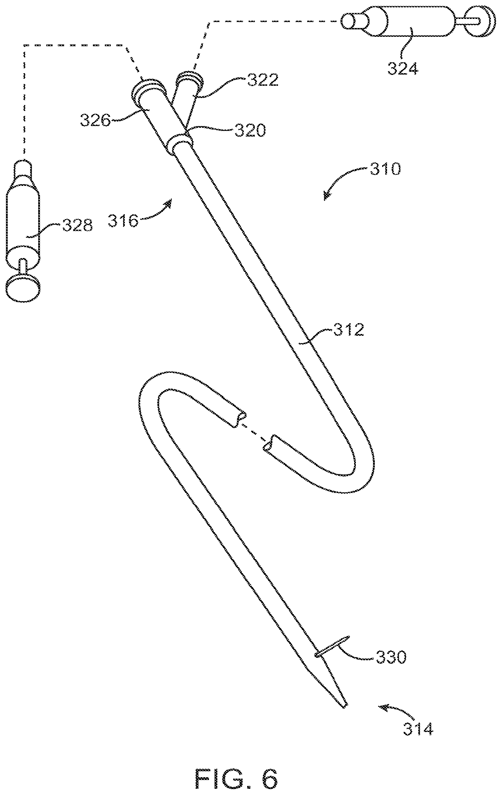

Referring now to Fig. 6, a needle injection catheter 310 constructed in accordance with the principles of the present invention comprises a catheter body 312 having a distal end 314 and a proximal 316. Usually, a guide wire lumen 313 will be provided in a distal nose 352 of the catheter, although over-the-wire and embodiments which do not require guide wire placement will also be within the scope of the present invention. A two-port hub 320 is attached to the proximal end 316 of the catheter body 312 and includes a first port 322 for delivery of a hydraulic fluid, e.g., using a syringe 324, and a second port 326 for delivering the pharmaceutical agent, e.g., using a syringe 328. A reciprocatable, deflectable needle 330 is mounted near the distal end of the catheter body 312 and is shown in its laterally advanced configuration in Fig. 6.

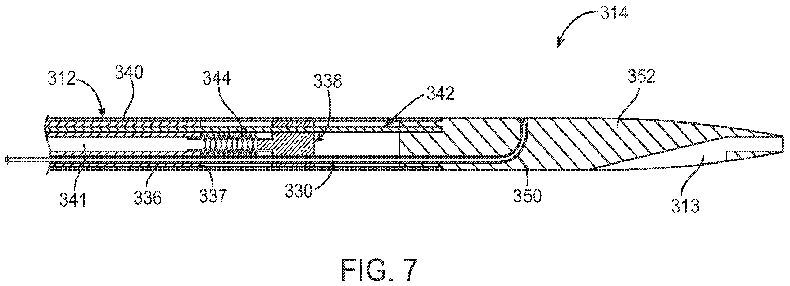

Referring now to Fig. 7, the proximal end 314 of the catheter body 312 has a main lumen 336 which holds the needle 330, a reciprocatable piston 338, and a hydraulic fluid delivery tube 340. The piston 338 is mounted to slide over a rail 342 and is fixedly attached to the needle 330. Thus, by delivering a pressurized hydraulic fluid through a lumen 341 tube 340 into a bellows structure 344, the piston 338 may be advanced axially toward the distal tip in order to cause the needle to pass through a deflection path 350 formed in a catheter nose 352.

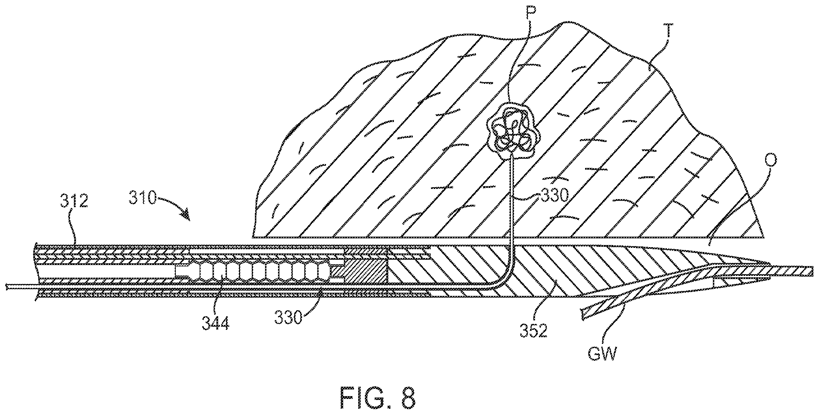

As can be seen in Fig. 8, the catheter 310 may be positioned in a coronary blood vessel BV, over a guide wire GW in a conventional manner. Distal advancement of the piston 338 causes the needle 330 to advance into luminal tissue T adjacent to the catheter when it is present in the blood vessel. The therapeutic or diagnostic agents may then be introduced through the port 326 using syringe 328 in order to introduce a plume P of agent in the cardiac tissue, as illustrated in Fig. 8. The plume P will be within or adjacent to the region of tissue damage as described above.

The needle 330 may extend the entire length of the catheter body 312 or, more usually, will extend only partially into the therapeutic or diagnostic agents delivery lumen 337 in the tube 340. A proximal end of the needle can form a sliding seal with the lumen 337 to permit pressurized delivery of the agent through the needle.

The needle 330 will be composed of an elastic material, typically an elastic or super elastic metal, typically being nitinol or other super elastic metal. Alternatively, the needle 330 could be formed from a non-elastically deformable or malleable metal which is shaped as it passes through a deflection path. The use of non-elastically deformable metals, however, is less preferred since such metals will generally not retain their straightened configuration after they pass through the deflection path.

The bellows structure 344 may be made by depositing by parylene or another conformal polymer layer onto a mandrel and then dissolving the mandrel from within the polymer shell structure. Alternatively, the bellows 344 could be made from an elastomeric material to form a balloon structure. In a still further alternative, a spring structure can be utilized in, on, or over the bellows in order to drive the bellows to a closed position in the absence of pressurized hydraulic fluid therein.

After the therapeutic material is delivered through the needle 330, as shown in Fig. 8, the needle is retracted and the catheter either repositioned for further agent delivery or withdrawn. In some embodiments, the needle will be retracted simply by aspirating the hydraulic fluid from the bellows 344. In other embodiments, needle retraction may be assisted by a return spring, e.g., locked between a distal face of the piston 338 and a proximal wall of the distal tip 352 (not shown) and/or by a pull wire attached to the piston and running through lumen 341.

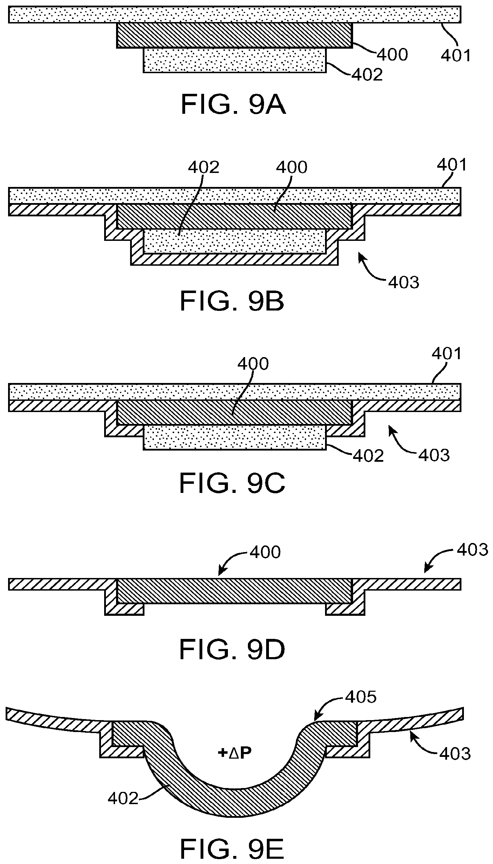

Figs. 9A-9E illustrate an exemplary process for fabricating a dual modulus balloon structure or anchored membrane structure in accordance with the principles of the present invention. The first step of the fabrication process is seen in Fig. 9A, in which a low modulus "patch", or membrane, material 400 is layered between removable (e.g. dissolvable) substrates 401 and 402. The substrate 401 covers one entire face of the patch 400, while the substrate 402 covers only a portion of the opposite face, leaving an exposed edge or border region about the periphery.

In Fig. 9B, a layer of a "flexible but relatively non-distensible" material 403 is deposited onto one side of the sandwich structure from Fig. 9A to provide a frame to which the low-modulus patch is attached. This material may be, for example, parylene N, C, or D, though it can be one of many other polymers or metals. When the flexible but relatively non-distensible material is parylene and the patch material is a silicone or siloxane polymer, a chemomechanical bond is formed between the layers, creating a strong and leak-free joint between the two materials. The joint formed between the two materials usually has a peel strength or interfacial strength of at least 0.05 N/mm², typically at least 0.1 N/mm², and often at least 0.2 N/mm².

In Fig. 9C, the "flexible but relatively non-distensible" frame or anchor material 403 has been trimmed or etched to expose the substrate material 402 so that it can be removed. Materials 401 and 402 may be dissolvable polymers that can be removed by one of many chemical solvents. In Fig. 9D, the materials 401 and 402 have been removed by dissolution, leaving materials 400 and 403 joined edge-to-edge to form the low modulus, or elastomeric, patch 400 within a frame of generally flexible but relatively non-distensible material 403.

As shown in Fig. 9E, when positive pressure +ΔP is applied to one side 405 of the structure, the non-distensible frame 403 deforms only slightly, while the elastomeric patch 400 deforms much more. The low modulus material may have a material modulus which is always lower than that of the high modulus material and is typically in the range from 0.1 to 1,000 MPa, more typically in the range from 1 to 250 MPa. The high modulus material may have a material modulus in the range from 1 to 50,000 MPa, more typically in the range from 10 to 10,000 MPa. The material thicknesses may range in both cases from approximately 1 micron to several millimeters, depending on the ultimate size of the intended product. For the treatment of most body lumens, the thicknesses of both material layers 402 and 403 are in the range from 10 microns to 2 mm.

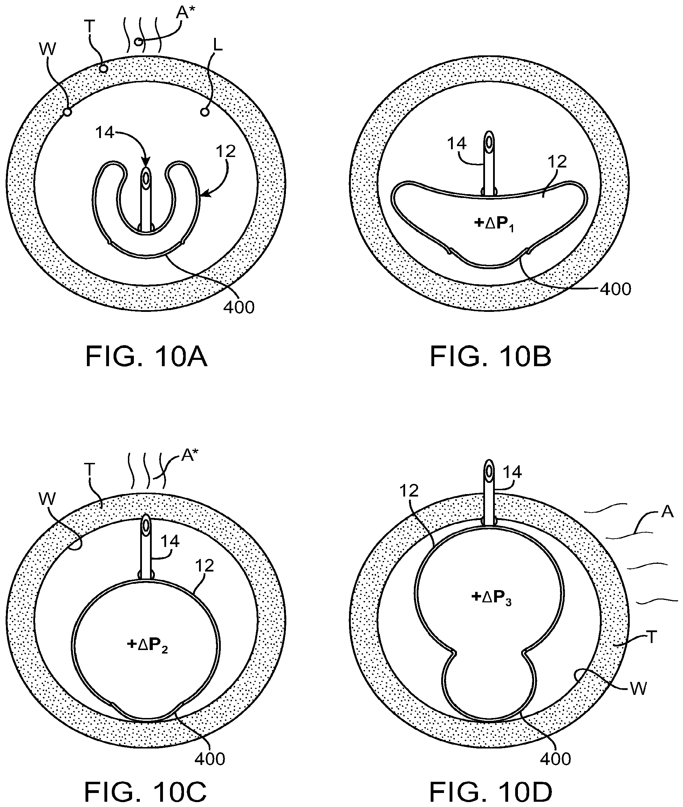

Referring to Figs. 10A-10D, the elastomeric patch of Figs. 9A-9D is integrated into the intraluminal catheter of Fig 1-5. In Fig 10A-D, the progressive pressurization of such a structure is displayed in order of increasing pressure. In Fig 10A, the balloon is placed within a body lumen L. The lumen wall W divides the lumen from periluminal tissue T, or adventitia A*, depending on the anatomy of the particular lumen. The pressure is neutral, and the non-distensible structure forms a U-shaped involuted balloon 12 similar to that in Fig. 1 in which a needle 14 is sheathed. While a needle is displayed in this diagram, other working elements including cutting blades, laser or fiber optic tips, radiofrequency transmitters, or other structures could be substituted for the needle. For all such structures, however, the elastomeric patch 400 will usually be disposed on the opposite side of the involuted balloon 12 from the needle 14.

Actuation of the balloon 12 occurs with positive pressurization. In Fig. 10B, pressure (+ΔP₁) is added, which begins to deform the flexible but relatively non-distensible structure, causing the balloon involution to begin its reversal toward the lower energy state of a round pressure vessel. At higher pressure +ΔP₂ in Fig. 10C, the flexible but relatively non-distensible balloon material has reached its rounded shape and the elastomeric patch has begun to stretch. Finally, in Fig. 10D at still higher pressure +ΔP₃, the elastomeric patch has stretched out to accommodate the full lumen diameter, providing an opposing force to the needle tip and sliding the needle through the lumen wall and into the adventitia. Typical dimensions for the body lumens contemplated in this figure are between 0.1 mm and 50 mm, more often between 0.5 mm and 20 mm, and most often between 1 mm and 10 mm. The thickness of the tissue between the lumen and adventitia is typically between 0.001 mm and 5 mm, more often between 0.01 mm and 2 mm and most often between 0.05 mm and 1 mm. The pressure +ΔP useful to cause actuation of the balloon is typically in the range from 0.1 atmospheres to 20 atmospheres, more typically in the range from 0.5 to 20 atmospheres, and often in the range from 1 to 10 atmospheres.

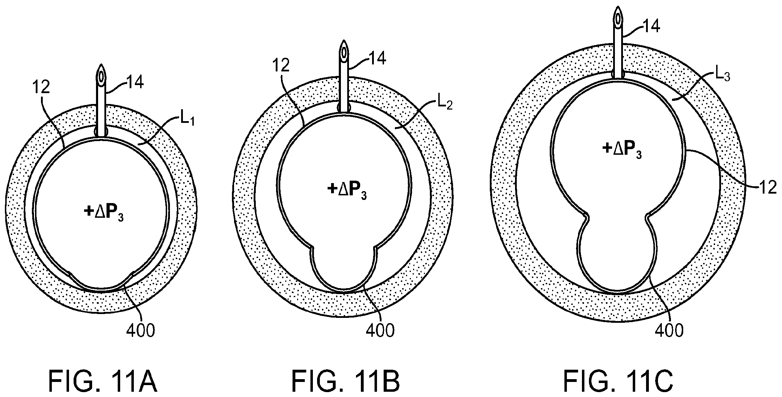

As illustrated in Figs. 11A-11C, the dual modulus structure formed herein provides for low-pressure (i.e., below pressures that may damage body tissues) actuation of an intraluminal medical device to place working elements such as needles in contact with or through lumen walls. By inflation of a constant pressure, and the elastomeric material will conform to the lumen diameter to provide full apposition. Dual modulus balloon 12 is inflated to a pressure +ΔP₃ in three different lumen diameters in Figs. 11A, 11B, and 11C. for the progressively larger inflation of patch 400 provides optimal apposition of the needle through the vessel wall regardless of diameter. Thus, a variable diameter system is created in which the same catheter may be employed in lumens throughout the body that are within a range of diameters. This is useful because most medical products are limited to very tight constraints (typically within 0.5 mm) in which lumens they may be used. A system as described in this invention may accommodate several millimeters of variability in the luminal diameters for which they are useful.

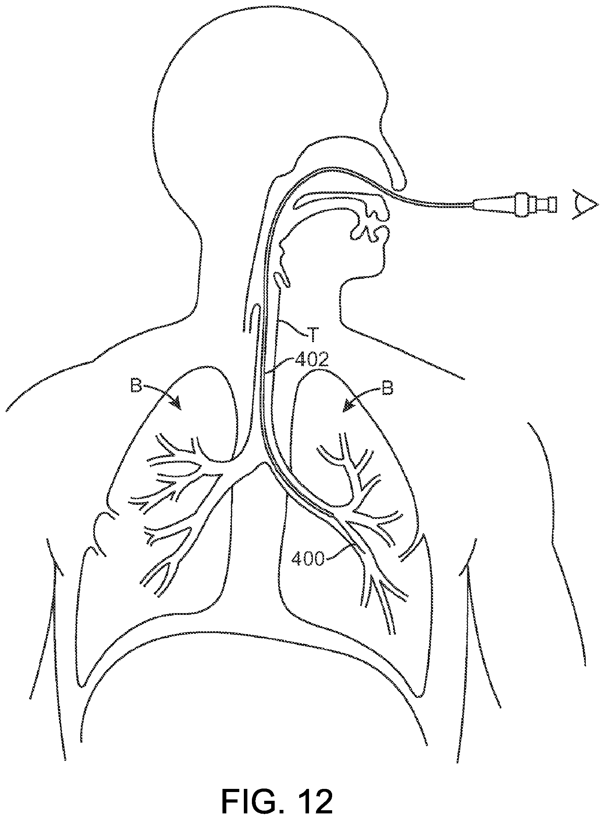

Referring now to Fig. 12, body lumens, conduits, vessels, and cavitary organs that may be treated in accordance with the present invention are present in the respiratory system. A catheter 400 may be introduced to an area of therapeutic interest as described above. At that position, a needle is deployed through the wall of the conduit and medication is delivered. Of particular interest to this invention, medication may be deployed to reduce hyperconstrictive smooth muscle in the lungs, for example in asthmatic patients or in patients who have had a bronchial carcinoma debulked, where the catheter is typically delivered through a bronchoscope 402 (Fig. 12). Also, anti-cancer therapeutic agents may be delivered into tumors that lie near or around the conduit through which the catheter may be introduced and deployed (i.e., in lung). Anti-cancer therapeutic agents may be delivered to tumors or tumor sites in the bronchus to debulk the tumors or prevent recurrence of the tumors at the tumor sites. A variety of bronchial tumors may be treated, for example, a debridable tumor of bronchial tissue in the airway, a lobar airway stenosis for which mechanical tumor debridement is not feasible, and an extrinsic airway stenosis for which mechanical tumor debridement is not feasible (because mechanical debridement would likely destroy the airway).

### EXPERIMENTAL STUDIES

Data from pre-clinical studies suggests that injecting paclitaxel into the bronchial adventitia using the balloon mounted injection needle described herein at a 0.5 mg/mL dose is safe. These studies demonstrate the ability to achieve high local concentrations of the therapeutic agent within the wall of the bronchus with no observable systemic or local parechymal toxicity.

Paclitaxel is a commercially available generic therapeutic agent with antitumor activity discovered in the 1970s. It is a clear, colorless, slightly viscous liquid, and the formulation of each one mL of solution contains 6 mg of active pharmaceutical ingredient paclitaxel. Paclitaxel is approved worldwide for treatment of non-small cell lung cancer, ovarian, and breast carcinoma, and AIDS-related Kaposi's sarcoma and has been extensively studied pre-clinically and clinically as a part of obtaining the requisite regulatory approvals. Typically, it is systemically administered via intravenous infusion over several hours at doses ranging between 135 and 175 mg/m² depending on the infusion duration. Adverse drug reactions associated with the systemic administration are well known.

Generic and proprietary paclitaxel formulations have been extensively studied not just for the approved indications, but also for other indications. Paclitaxel is an antimicrotubule agent that promotes the assembly of microtubules from tubulin dimers and stabilizes microtubules by preventing depolymerization. This stability results in the inhibition of the normal dynamic reorganization of the microtubule network that is essential for vital interphase and mitotic cellular functions. In addition, paclitaxel induces abnormal arrays or "bundles" of microtubules throughout the cell cycle and multiple asters of microtubules during mitosis. As a result, paclitaxel inhibits normal cell proliferation.

Paclitaxel can be used in the treatment of different solid tumors. Paclitaxel alone (generic and proprietary formulations) is used as a first and second line treatment against ovarian, breast, lung and other types of carcinoma. It is also used in combination with carboplatin and other agents.

Systemic administration of paclitaxel can lead to toxicities to normal tissues. Paclitaxel is a chemotherapeutic agent, but as such it could cause toxic effects on peripheral nerves with different severities. Peripheral neuropathy could be dose-limiting side effect.

Paclitaxel has been extensively studied as part of obtaining marketing approval in the USA (NDA 020262) and world-wide and it is being currently investigated for other indications and in combination with newly discovered agents (NCT00021060). As of June 2013, there are over 1900 studies listed on www.clinicaltrials.gov involving paclitaxel, of which 396 are investigating paclitaxel in lung cancer, and of them 71 are currently recruiting patients. Thirty two (32) of the currently recruiting studies are enrolling patients with stage IV lung cancer. This demonstrates a clinical need for paclitaxel as a therapeutic agent for lung cancer. At the same time, there is a vast safety database for paclitaxel that has been accumulated over the years.

In the pre-clinical studies performed, paclitaxel was delivered using the Blowfish Transbronchial Micro-Infusion Catheter available from Mercator Medsystems of San Leandro, CA, which is commercially available and intended to deliver therapeutic and diagnostic agents that are indicated or labeled for airway, tracheal, or bronchial delivery into selected and sub-selected regions of the airway tree.

### Generic Paclixtaxel (Taxol) Studies

A GLP study with 10 pigs and two paclitaxel concentrations was conducted. Injections of saline (placebo) or 0.4 and 1.5 mg/mL paclitaxel (PTX) to the bronchial adventitia of Yorkshire pigs using a Mercator Blowfish Transbronchial Micro-Infusion Catheter were well-tolerated by the animals under the conditions of this study. Other than a transient reaction to PTX or excipient (Cremophor EL) for a single animal administered 1.5 mg/mL PTX infusions, there were no other infusion or PTX related abnormalities in the clinical observations, body weights, and clinical pathology results. Microscopic evaluation after 28 days was associated with favorable local tissue responses that were comparable between the saline control, low doses (0.5 mg/mL) and high does (1.5 mg/mL) PTX groups. Injury was absent to negligible, and comparable between Treated and Control groups. Epithelial loss was negligible across groups, and fibrin/luminal hemorrhage/thrombus absent to negligible. Inflammation associated with treatment was also absent to negligible, and the minimal lymphocytes present were considered part of normal BALT. One individual female animal from the Placebo Control group exhibited multifocal pneumonia and mild bronchial inflammation that was unrelated to PTX, and may have been caused by bronchoscopic procedure alone or due to an infectious inhalant or non-infectious aspiration etiology.

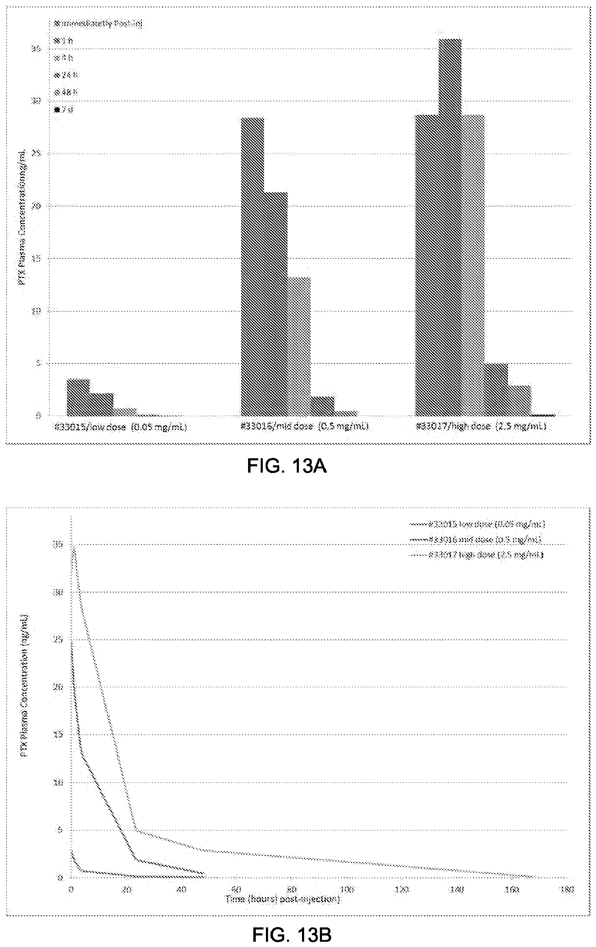

As shown in Figs. 13A and 13B, PTX was not present in the plasma of control animals, but was measured in plasma samples of both drug groups out to 120 hours (5 days). No PTX was detected at 28 days post infusion in any animal. The AUC_{(0-5d)} was calculated to be 122 ± 15 ng^{∗}h/mL for the 0.5 mg group (with an average total dose of 5.2 ± 0.3 mg across 10.3 ± 0.6 infusions) and 320 ± 61 ng*h/mL for the 1.5 mg group (with a total dose of 15 mg in each animal). These AUC_{(0-5d)} levels meet the acceptance criterion established by the paclitaxel package insert, which describes an AUC(0-∞) of 6,300 ng^{∗}h/mL for a 135 mg/m² dose administered over 24 hours.

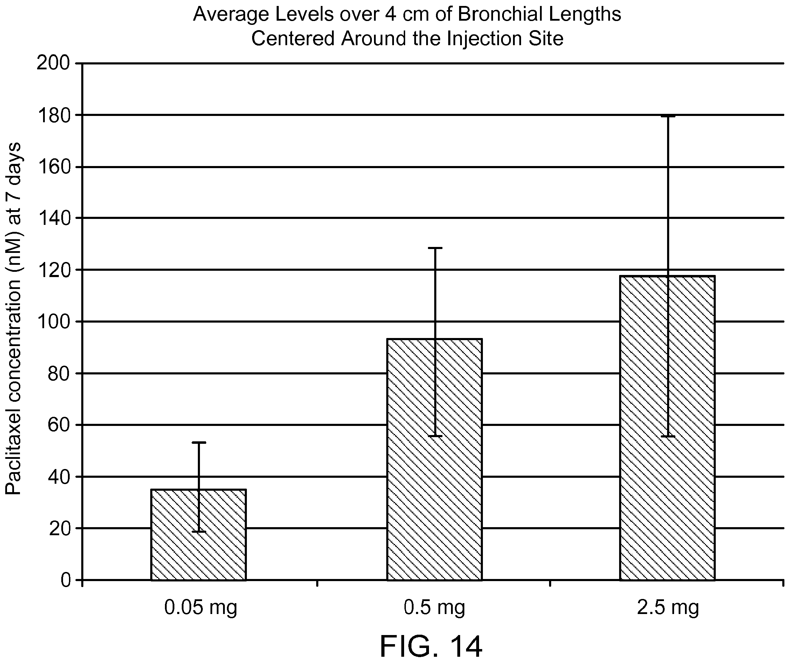

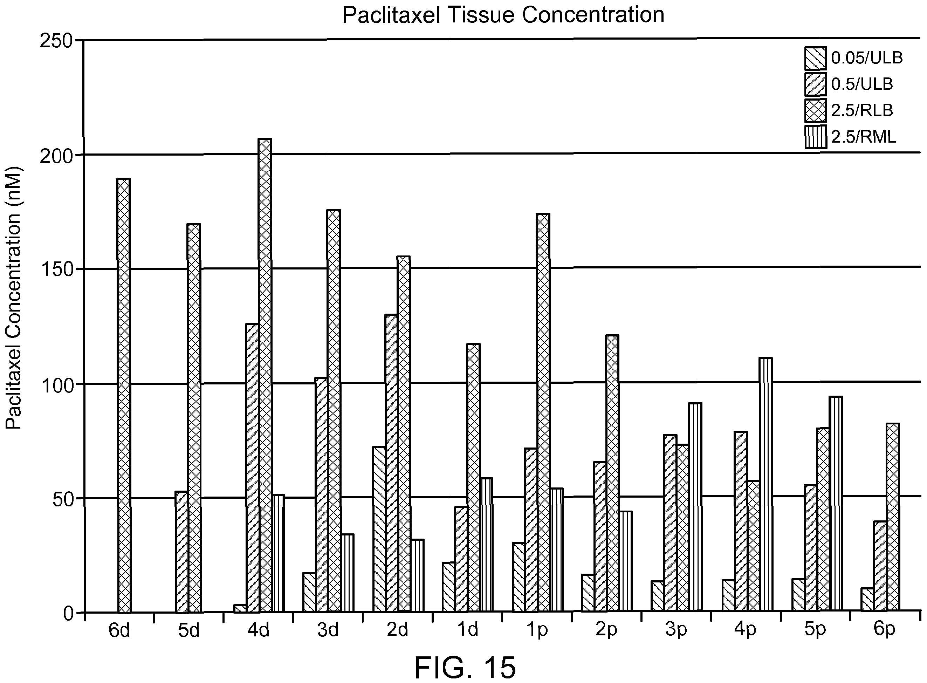

Paclitaxel Tissue Concentrations: Bronchial tissue was collected for tissue PTX analysis. Fig. 14 shows the average paclitaxel concentration (nM) at 7 days over 4 cm of bronchial tissue centered around the injection site (2 cm distal and 2 cm proximal). Average paclitaxel concentrations in the first two distal and first two proximal segments in each dose group (low, mid and high) were 35 ±15 nM (range from 14.7 nM to 50.4 nM), 86 ± 33 nM (ranging from 26.7 nM to 122.1 nM) and 94 ± 67 nM (ranging from 47.1 nM to 141.4 nM), respectively. Since the drug was present in these concentrations at 7 days, these drug tissue levels are above the 10 - 30 nM values reported in the literature as effective if present for 96 hours in suppressing cancer cell lines such as H358 an H460 [Zou et al., 2004]. In each dose group, there was one injection site for which all collected distal and proximal samples were analyzed (Figure 15). For segments in Figure 15 in which no column is present, it is not a zero measurement, but a lack of tissue sample corresponding to the omitted columns.

From a review of the tissue results in conjunction with the plasma concentration data, it can be concluded that paclitaxel was present in bronchial tissue of the 0.5 and the 1.5 mg/mL paclitaxel groups even after 28 days, while at the same time the local tissue reaction was mild to negligible in all groups.

### Histopathology and Drug Tissue Concentration One Week After Paclitaxel Delivery to Porcine Bronchial Adventitia In Vivo:

After 7 days in porcine model, treatment of bronchial wall using the Mercator Blowfish Transbronchial Micro-Infusion Catheter for paclitaxel delivery was associated with evidence of a lymphocytic response and mild inflammation at doses of 0.05 mg per injections site and 0.5 mg per injection site however these doses were not associated with evidence of damage. Specifically there was no evidence of luminal thrombus bronchial injury and minimal epithelial loss.

At the highest dose tested (2.5 mg/mL, i.e. 5 mg per injection site), there was multifocal marked subacute necrosis of bronchial cartilage, peribronchial tissue and pulmonary parenchyma, with moderate associated inflammation. Mean bronchial injury in this group was moderate (i.e. lacerated smooth muscle), while luminal thrombus and epithelial loss were overall minimal.

Plasma paclitaxel concentrations decreased over time. In the low (0.05 mg/site, i.e. 0.65 mg total paclitaxel injected) and medium (0.5 mg/site, i.e. 6.5 mg total paclitaxel injected) dose pigs they were below the method's Limit of Quantitation (LOQ=0.03 ng/mL) at 7 days. In the high dose animal (5 mg paclitaxel per site and total of 25 mg paclitaxel injected), even at 7 days, the paclitaxel plasma concentration was at detectible levels (at 0.124 ng/mL).

Paclitaxel plasma concentration area under the curve (AUC): AUCₗₐₛₜ for the low dose (0.65 mg of total paclitaxel) and medium dose (6.5 mg of total paclitaxel) was 18.46 ng^{∗}h/mL and 255.5 ng^{∗}h/mL, respectively and AUCₗₐₛₜ for the high dose pig was 740.40 ng^{∗}h/mL. These values are lower than what has been reported for IV administered paclitaxel in the FDA approved Package Insert for Taxol (NDA 020262): AUC_{(0-∞)} between 6,300 and 15,007 ng^{∗}h/mL. As the local dosing resulted in lower systemic exposure than currently approved doses, no new systemic toxic effects are anticipated.

It is noted that concentrations of around 20 nM of paclitaxel were found to be effective in suppressing cancer cell lines such as H358 and H460 according to various studies in the literature. Average paclitaxel concentrations in the first two distal and first two proximal segments in each dose group (low, mid and high) were 35 ± 15 nM, 86 ± 33 nM and 94 ± 67 nM, respectively. Since the drug was present in these concentrations at 7 days, these drug tissue levels are likely above the 10 - 30 nM values reported in the literature as effective if present for 96 hours in suppressing cancer cell lines such as H358 an H460.

The data above indicate that it was safe to deliver paclitaxel at 0.05 and 0.5 mg/mL dose levels using the Blowfish Catheter. Injecting 2 mL of paclitaxel at 2.5 mg/mL, i.e. 5 mg paclitaxel per site was found to cause local adverse reactions that could be considered dose-limiting toxicities. Plasma paclitaxel levels drop below the LOQ of the method within 7 days for the low and mid dose but are sustained above LOQ for the high dose to 7 days. The tissue paclitaxel concentration data indicate that there is sufficient drug in the bronchial adventitia at cancer inhibiting levels, yet there were no observed systemic toxicities in any of the studied concentrations.

### Abraxane® Studies

Studies using 0.5 mg/mL Abraxane® (a proprietary paclitaxel formulation) instead of Taxol, i.e. generic paclitaxel, formulated with Cremophor EL were conducted. These 1-, 7- and 20-day studies also indicated that injecting paclitaxel active ingredient into the bronchial wall was safe and resulted in chemotherapeutic concentrations at all time-points analyzed. The local tissue reaction to the infusion of paclitaxel was negligible, and there were no injuries or epithelial loss in paclitaxel injected segments. Focal findings of inflammation and Hemorrhage/Fibrin/ Thrombus were at worst mild on average. No injury or epithelial loss was found beyond 1 day in paclitaxel injected segments.

### Study Conclusions

These studies demonstrate that: (1) Blowfish Catheter injection is safe; (2) paclitaxel injections into the bronchial wall at 1.5 mg/mL dose or less are safe; (3) tissue levels of paclitaxel are maintained at cancer-inhibiting levels to 7 days for generic paclitaxel and to 20 days for Abraxane®. Thus, Applicants believe paclitaxel is suitable for the treatment of non-small cell lung cancer by localized delivery in the airway wall with a proposed dose of 1.5 mg/mL, with a total of 1.5 mg/subject.

## Claims

1. A cytotoxic, cytostatic, or anti-neoplastic agent for use in maintaining patency in a bronchus of a patient, wherein the cytotoxic, cytostatic, or anti-neoplastic agent is to be delivered in a therapeutically beneficial amount effective to limit recurrent bronchial occlusion into one or more of a bronchial wall, submucosa, media, or adventitia of the bronchus, and wherein the agent is delivered through a needle positioned through a wall of the bronchus so that an aperture of the needle is positioned at or beyond the bronchial adventitia, wherein the agent for delivery has a concentration in the range of 0.5 mg/mL to 1.5 mg/mL.

2. The agent for use of claim 1, wherein the amount of the agent is delivered to a site at or adjacent a cancerous tumor, and wherein optionally the cancerous tumor comprises a bronchia carcinoma, granuloma, fibrosis, or benign or malignant structure or narrowing.

3. The agent for use of claim 2, wherein the amount of agent is delivered to a site at or adjacent a cancerous tumor, wherein the cancerous tumor has been debulked prior to delivery of the agent.

4. The agent for use of claim 2 or 3, wherein the delivered amount of agent is effective to prevent the recurrence of the cancerous tumor.

5. The agent for use of any one of claims 1 to 4, wherein the needle comprises a 35 to 45 gauge needle.

6. The agent for use of any one of claims 1 to 5, wherein the penetration of the tissue by the agent is confirmed by imaging either the agent mixed with a diagnostic agent or by delivery of a diagnostic agent prior to the delivery of the agent.

7. The agent for use of any one of claims 1 to 6, wherein the agent is delivered through a catheter advanced into the bronchus and the catheter is positioned adjacent a target region of the bronchial wall and adventitia before delivery of the agent.

8. The agent for use of claim 7, wherein the catheter comprises an expandable element disposed on a distal end thereof and a needle disposed on the expandable element, wherein the expandable element is expandable to cause the needle to puncture the target region of the bronchial wall, submucosa, media, or adventitia before delivery of the agent.

9. The agent for use of claim 8, wherein the expandable element comprises an inflatable balloon, wherein optionally:
(a) the inflatable balloon is inflatable with 2 atmospheres of pressure without damaging the bronchus; and/or
(b) the inflatable balloon is inflated with air or saline.

10. The agent for use of any one of claims 1 to 9, wherein the agent comprises paclitaxel.

11. The agent for use of claim 10, wherein the agent comprises Abraxane®.

12. The agent for use of claim 10 or claim 11, wherein no observable systemic or local parenchymal toxicity is associated with delivery of the agent.

## Patentansprüche

1. Cytotoxisches, cytostatisches oder antineoplastisches Mittel zur Verwendung bei der Aufrechterhaltung der Durchgängigkeit in einem Bronchus eines Patienten, wobei das cytotoxische, cytostatische oder antineoplastische Mittel in einer therapeutisch nützlichen Menge mit Wirkung zur Begrenzung des rezidivierenden Bronchusverschlusses in eine oder mehr von einer bronchialen Wand, Submucosa, Media oder Adventitia des Bronchus abzugeben ist, und wobei das Mittel über eine Nadel abgegeben wird, die durch eine Wand des Brochus derart positioniert wird, dass eine Öffnung der Nadel an der oder weiter weg von der Adventitia der Bronchien positioniert wird, wobei das Mittel zur Abgabe eine Konzentration im Bereich von 0,5 mg/ml bis 1,5 mg/ml aufweist.

2. Mittel zur Verwendung nach Anspruch 1, wobei die Menge des Mittels an einer Stelle an einen oder benachbart an einen kanzerösen Tumor abgegeben wird, und wobei der kanzeröse Tumor gegebenenfalls ein(e) Bronchialkarzinom, -granulom, -fibrose oder benigne oder maligne Struktur oder Verengung umfasst.

3. Mittel zur Verwendung nach Anspruch 2, wobei die Menge des Mittels an einer Stelle an einen oder benachbart an einen kanzerösen Tumor abgegeben wird, wobei der kanzeröse Tumor vor Abgabe des Mittels mittels Debulking reduziert worden ist.

4. Mittel zur Verwendung nach Anspruch 2 oder 3, wobei die abgegebene Menge des Mittels zur Prävention eines Rezidivs des kanzerösen Tumors wirksam ist.

5. Mittel zur Verwendung nach einem der Ansprüche 1 bis 4, wobei die Nadel eine Nadel von 35 bis 45 Gauge umfasst.

6. Mittel zur Verwendung nach einem der Ansprüche 1 bis 5, wobei die Durchdringung des Gewebes mit dem Mittel mittels Bildgebung, entweder des Mittels gemischt mit einem diagnostischen Mittel oder durch Abgabe eines diagnostischen Mittels vor Abgabe des Mittels, bestätigt wird.

7. Mittel zur Verwendung nach einem der Ansprüche 1 bis 6, wobei das Mittel über einen in den Bronchus vorgeschobenen Katheter abgegeben wird und der Katheter vor Abgabe des Mittels benachbart an einen Zielbereich der bronchialen Wand und Adventitia positioniert wird.

8. Mittel zur Verwendung nach Anspruch 7, wobei der Katheter ein auf einem distalen Ende davon angeordnetes expandierbares Element umfasst und eine Nadel auf dem expandierbaren Element angeordnet ist, wobei das expandierbare Element expandierbar ist, um mit der Nadel vor Abgabe des Mittels die Punktion der Zielregion der bronchialen Wand, Submucosa, Media oder Adventitia zu bewirken.

9. Mittel zur Verwendung nach Anspruch 8, wobei das expandierbare Element einen aufblasbaren Ballon umfasst, wobei gegebenenfalls:
(a) der aufblasbare Ballon mit einem Druck von 2 Atmosphären ohne Schädigung des Bronchus aufblasbar ist; und/oder
(b) der aufblasbare Ballon mit Luft oder einer Kochsalzlösung aufgeblasen wird.

10. Mittel zur Verwendung nach einem der Ansprüche 1 bis 9, wobei das Mittel Paclitaxel umfasst.

11. Mittel zur Verwendung nach Anspruch 10, wobei das Mittel Abraxane® umfasst.

12. Mittel zur Verwendung nach Anspruch 10 oder Anspruch 11, wobei mit Abgabe des Mittels keine beobachtbare systemische oder lokale Parenchymtoxizität assoziiert ist.

## Revendications

1. Agent cytotoxique, cytostatique ou antinéoplasique, destiné à une utilisation dans le maintien de la perméabilité dans une bronche d'un patient, l'agent cytotoxique, cytostatique ou antinéoplasique devant être administré dans une quantité apportant un bénéfice thérapeutique, efficace pour limiter une occlusion bronchique récidivante dans une ou plusieurs d'une paroi bronchique, d'une sous-muqueuse, d'une tunique moyenne ou d'une adventice de la bronche, et l'agent étant administré par l'intermédiaire d'une aiguille positionnée à travers une paroi de la bronche de telle sorte qu'une ouverture de l'aiguille soit positionnée dans ou au-delà de l'adventice bronchique, l'agent à administrer ayant une concentration de 0,5 mg/ml à 1,5 mg/ml.

2. Agent destiné à une utilisation selon la revendication 1, la quantité de l'agent étant administrée dans un site situé dans ou adjacent à une tumeur cancéreuse, et, optionnellement, la tumeur cancéreuse comprenant un carcinome bronchique, un granulome, une fibrose, ou une structure bénigne ou maligne ou un rétrécissement.

3. Agent destiné à une utilisation selon la revendication 2, la quantité d'agent étant administrée dans un site situé dans ou adjacent à une tumeur cancéreuse, la tumeur cancéreuse ayant fait l'objet d'une cytoréduction avant l'administration de l'agent.

4. Agent destiné à une utilisation selon la revendication 2 ou 3, la quantité administrée de l'agent étant efficace pour prévenir la récidive de la tumeur cancéreuse.

5. Agent destiné à une utilisation selon l'une quelconque des revendications 1 à 4, l'aiguille comprenant une aiguille de 35 à 45 gauges.

6. Agent destiné à une utilisation selon l'une quelconque des revendications 1 à 5, la pénétration de l'agent dans le tissu étant confirmée en visualisant par imagerie soit l'agent mélangé avec un agent de diagnostic, soit un agent de diagnostic administré avant d'administrer l'agent.

7. Agent destiné à une utilisation selon l'une quelconque des revendications 1 à 6, l'agent étant administré par l'intermédiaire d'un cathéter que l'on fait avancer dans la bronche et le cathéter étant positionné de façon adjacente à une région cible de la paroi bronchique et de l'adventice avant l'administration de l'agent.

8. Agent destiné à une utilisation selon la revendication 7, le cathéter comprenant un élément expansible disposé sur une extrémité distale dudit cathéter et une aiguille disposée sur l'élément expansible, l'élément expansible étant expansible pour amener l'aiguille à percer la région cible de la paroi bronchique, de la sous-muqueuse, de la tunique moyenne ou de l'adventice avant l'administration de l'agent.

9. Agent destiné à une utilisation selon la revendication 8, l'élément expansible comprenant un ballon gonflable, optionnellement :
(a) le ballon gonflable pouvant être gonflé à une pression de 2 atmosphères sans endommager la bronche ; et/ou
(b) le ballon gonflable étant gonflé avec de l'air ou avec une solution saline.

10. Agent destiné à une utilisation selon l'une quelconque des revendications 1 à 9, l'agent comprenant le paclitaxel.

11. Agent destiné à une utilisation selon la revendication 10, l'agent comprenant l'Abraxane®.

12. Agent destiné à une utilisation selon la revendication 10 ou la revendication 11, aucune toxicité systémique ou parenchymateuse locale observable n'étant associée à l'administration de l'agent.
